# EUROPEAN PATENT SPECIFICATION

(11) **EP 2 836 135 B1**
(45) Date of publication and mention of the grant of the patent: **25.10.2017**
(21) Application number: 13718882.7
(22) Date of filing: 12.04.2013
(51) Int. Cl.: A61B 17/17, A61F 2/46

(54) **DEVICE FOR USE IN FACILITATING ALIGNMENT OF AN ACETABULAR COMPONENT**
VORRICHTUNG ZUR ERLEICHTERUNG DER AUSRICHTUNG EINER HÜFTPFANNENKOMPONENTE
DISPOSITIF DESTINÉ À ÊTRE UTILISÉ POUR FACILITER L'ALIGNEMENT D'UN COMPOSANT ACÉTABULAIRE

(30) Priority: 12.04.2012 GB 201206493
(43) Date of publication of application: 18.02.2015
(73) Proprietor: Imperial Innovations Limited, London SW7 2PG (GB)
(72) Inventor: CLARKE, Susannah G., London SW18 5AY (GB); COBB, Justin P., London W4 2PS (GB)
(74) Representative: CSY St Albans
(86) International application number: PCT/GB2013/050951
(87) International publication number: WO 2013/153401

(56) References cited:
- EP-A2- 2 168 507
- WO-A2-2012/024288
- US-A1- 2012 041 445

## Description

The invention relates to a device for use in facilitating alignment of an acetabular component relative to the pelvis of a subject. The invention also relates to a kit for such a device. Acetabular cups form the socket part of the 'ball and socket' hip replacement or hip resurfacing. An acetabular cup is a hemi-spherically shaped, or near hemi-spherical shaped, shell implant which is implanted into the pelvis and into which a spherical femoral head implant can articulate. There is much recent literature discussion on the importance of acetabular cup orientation, positioning and sizing, with clinical analyses showing that poor cup position correlates with failure.

Implant retrieval studies from multiple centres have demonstrated significant correlations between acetabular cup positioning and accelerated wear leading to early failure. A direct link has therefore been made between the orientation, positioning and/or sizing of the acetabular cup and early failure; this has been substantiated by experimental research. Cemented acetabular cup implants do not appear to be as sensitive to cup positioning as cementless implants. The same methods for cup orientation, positioning and sizing are used for both implant types. Cup orientation and position is defined in terms of inclination angle (angle from the transverse plane) and version angle (angle from coronal plane) and cup centre position. Research shows that there is a consistent level of cup inclination over which potential for accelerated wear is substantially increased, but below which impingement (unintended clashing of bone and implant during movement) may occur. The optimal level of inclination depends upon the shape of the prosthetic cup. Those cups which are close to hemispherical should optimally be inclined at 45°, while cups which are substantially less than hemispherical (such as large diameter cups suitable for hip resurfacing) need to be less inclined, at closer to 40° (this is to ensure that the wear patch does not reach the edge of the cup, precipitating edge loading). For each type of device, there is an accepted zone of safety for inclination and version, but the optimal position varies from patient to patient to ensure that neither edge loading nor impingement occurs.

Cup position is defined in relation to the hip centre. The hip centre is rarely entirely normal prior to surgery. Commonly it is either too deep or too superficial in the horizontal or axial plane. The hip centre may also be too anterior and too high, in hips with shallow sockets, or too posterior and too high in hips with sockets that are too deep. The prosthetic cup should be inserted at an optimal position, most importantly in terms of depth, to prevent problems such as the cup rim rubbing on soft tissues and/or impingement.

There is a current NHS initiative for 'Enhanced Recovery'. This programme is aimed at speeding up patient recovery post-operatively. Minimally invasive surgery describes surgery conducted through a small incision with minimal disruption to surrounding soft tissues. The reduced trauma and blood loss aid faster patient recovery and result in smaller wound size: a concern for the patient. This type of surgery is a growing trend. In the vast majority of hip replacements, acetabular cups implants are oriented by eye. The small incision associated with minimally invasive surgery obscures the visual location of the acetabular cup, increasing the potential for mal-position.

Current methods of cup positioning range from low-tech to high-tech, and are discussed below. The low-tech methods (visual alignment, simple mechanical alignment guides) are those currently used in the majority of surgeries and cannot currently avoid cup mal-position. The high-tech methods are computer-guided navigation and patient-matched templates. These methods are accurate but are costly and require CT-data, which is not routinely obtained in most hospitals.

### VISUAL ALIGNMENT / BASIC MECHANICAL ALIGNMENT GUIDE

By far the most prevalent technique of cup positioning is visual alignment. Orthopaedic companies do provide simple guides which connect to the impaction and reaming instruments, but as these guides rely on global frames of reference such as the horizontal plane of the room and the length of the bed, they can only offer a loose, non-specific indication of cup orientation, and no information on cup centre location.

### COMPUTER-GUIDED NAVIGATION

1% of the hip replacement procedures undertaken in 2010, reported in the National Joint Registry of England and Wales, used computer-guided navigation; this indicates the low level of take up. Two types of computer-guided navigation exist: that which relies on CT-based imaging, and that which does not (subject bone anatomy is referenced intra-operatively). Both systems require substantial capital investment, and the CT-based system requires long-term increased costs owing to the required CT-scan. Such investment is only ever likely to occur in high-volume centres. CT-scanning the abdomen also exposes the patient to undesirable levels of radiation.

### PATIENT-MATCHED ALIGNMENT GUIDES

Patient-matched alignment guides use patient CT-data to design a subject-specific alignment guide which can ideally place the cup for each specific patient. The process requires CT-data (with associated costs and radiation risk) and also requires the service of an engineer to design and make (e.g. by 3D printing) the patient-matched guide; this adds considerable lead time to the surgery in terms of delay between proposed plan and finalised guide.

There is therefore a need for a device which is inexpensive and not CT-reliant, yet which is more accurate than existing low-tech techniques. Documents WO 2012/024288 A2 and US 2012/0041445 A1 disclose devices according to the preamble of claim 1. According to the invention there is provided a device for use in facilitating alignment of an acetabular component relative to the pelvis of a subject, as defined in claim 1. The acetabular rim is the prominent border that forms a boundary to the acetabulum, the acetabular rim being uneven and having a series of prominences and depressions. An acetabular component can be aligned to the orientation and/or position defined by the device directly (e.g. by aligning the acetabular component with at least part of the device when installed in the subject), or indirectly (e.g. by using the device to install a guide to which the acetabular component can be aligned).

The device body can be any part of the device that couples the at least a first locator with the rest of the device, and the device body can be any suitable shape. Preferred embodiments are defined by the dependent claims. The device is installable at an implant site in a subject's pelvis and is capable of autolocating to find at least one reference point on the acetabular rim from which optimal cup position can be defined. The natural landmark is a bony landmark that can easily be identified by the user (visually or by palpation). The natural landmark serves only to locate the device to the mounting location, not to define a reference line or reference plane from which optimal cup position can be defined.

The device is a universal device rather than a subject-specific device that is configured for use with a single, specific subject. In other words, the device can be used as an acetabular component alignment guide for a plurality of subjects. Prior art universal alignment guides locate to points distant from the acetabulum, as it is necessary to locate to landmarks that can be identified by the user visually or by palpation, whereas specific points on the acetabular rim cannot be identified visually or by palpation. However, the device of the present invention can locate to specific reference points on the acetabular rim, accurately and repeatably. By means of the arrangement of the alignment reference element and the first locator, the device can be aligned to a predetermined identifiable natural landmark other than on the acetabular rim to locate the first locator to a predetermined reference point on the rim. The acetabular component to be positioned in the subject using the device can be a trial cup, reamer or acetabular cup implant. The device provides a low cost navigation system for use in hip replacement procedures.

The device does not contain any surface portions that have been designed to conform exactly to the shape and contours of a corresponding portion of a particular subject's pelvic anatomy, unlike prior art patient-matched devices. As such, the device of the present invention can be placed in a plurality of spatial orientations relative to a subject's pelvis. The alignment reference element allows the device to be aligned such that the locators locate to the corresponding predetermined mounting locations, whereby the device can be installed in an orientation and/or position relative to the subject from which optimal cup orientation and/or position can be defined.

The device can be used in traditional surgery and minimally invasive surgery where the visual location of the acetabular cup is obscured. The device does not require a CT-scan of the subject in order to locate the locator to the corresponding mounting location, unlike expensive prior art patient-matched systems.

The invention also provides a kit of parts that, when assembled, provides a device as described above in accordance with any aspect of the invention.

The term patient as used herein may refer to any human or animal subject.

The term proximal as used herein may mean located near or toward the centre of the subject's body when the device is installed and/or situated toward the point of attachment. The term distal as used herein may mean located away from the centre of the body when the device is installed and/or situated away from the point of attachment.

Preferred embodiments of the present invention will now be more particularly described by way of example only with reference to the accompanying drawings, wherein:
Figures 1A and 1B show differing views of an acetabular cup implanted in a pelvis;
Figure 1C is a diagrammatic view of an acetabular cup installed in a pelvis indicating how orientation of the cup is defined by inclination angle I and version angle V;
Figure 1D is a diagrammatic view of a hip joint indicating the centre edge angle of the acetabulum, Vce;
Figure 1E is a view of a pelvic bone indicating the obturator externus tendon groove natural landmark;
Figure 2 is a graph showing the correlation between Vce angle and inferred inclination; Figures 3A to 15 show a device according to a first embodiment of the invention;
Figures 3A and 3B show perspective views of the first portion of the device, showing adjustment of the superior locator between;
Figure 4 shows the first portion of the device attached to an introducer with cup-shaped component attached;
Figure 5 shows the second portion of the device being assembled to the device of Figure 4;
Figure 6 shows the second portion of the device being further slidably mounted onto the first portion;
Figure 7 shows the assembly of Figure 6 initially introduced to a subject's left acetabulum;
Figure 8 shows the assembly of Figure 7 with a device alignment pin installed;
Figure 9 shows the assembly of Figure 8 having been tilted such that the locators rest on the acetabular rim;
Figure 10 shows a further view of the assembly of Figure 9;
Figure 11 shows the assembly of Figure 9, the device alignment pin having been removed and a pair of acetabular component alignment pins having been installed;
Figure 12 shows the acetabulum, the device assembly having been removed, leaving the acetabular component alignment pins installed in the subject;
Figure 13 shows a cup guide installed on the acetabular component alignment pins;
Figure 14 shows a further view of the cup guide of Figure 13;
Figure 15 shows an acetabular cup implant installed in the subject beneath the cup guide, with the cup guide being used as a post-impaction orientation and positioning check;
Figure 16 shows an axial underside view of the device of Figure 6, without the cup-shaped component or introducer assembled to the device;
Figure 17 shows a graph of radiographic version and inclination achieved for theoretical positioning in 65 sample subjects;
Figures 18 to 23 show a second embodiment of the invention; Figure 18 shows a front view of the device of the second embodiment;
Figure 19 shows a view of the device of Figure 18 from another side, hidden features being shown in dotted lines;
Figure 20 shows a back view of the device of Figure 18;
Figure 21 shows a perspective view of the device of the device of Figure 18;
Figure 22 shows a top view of the device of Figure 18;
Figure 23 shows a bottom view of the device of Figure 18;
Figures 24 to 28 show a third embodiment of the invention; Figure 24 shows a left side view of a device of the third embodiment;
Figure 25 shows a front view of the device of Figure 24;
Figure 26 shows a right side view of the device of Figure 24;
Figure 27 shows a perspective view of the device of Figure 24;
Figure 28 shows a bottom view of the device of Figure 24.

The present embodiments represent currently the best ways known to the applicant of putting the invention into practice. But they are not the only ways in which this can be achieved. They are illustrated, and they will now be described, by way of example only.

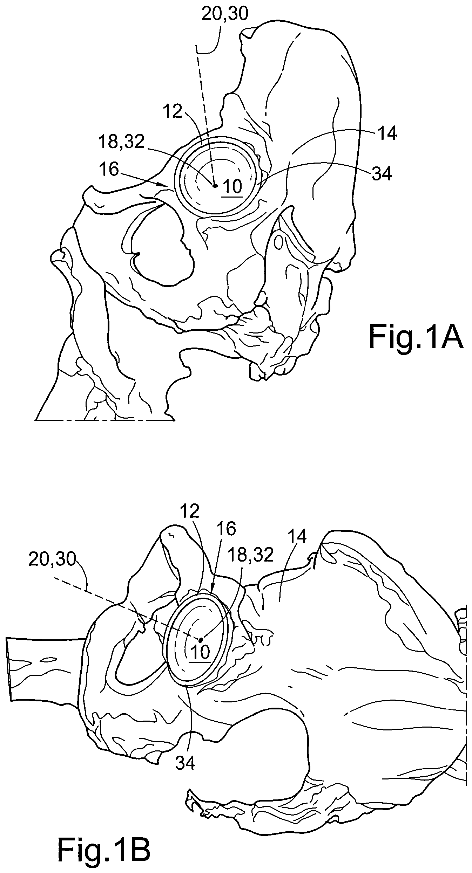

Referring to Figures 1A and 1B, an acetabular component is illustrated in the form of an acetabular cup 10 shown implanted in place in an acetabulum 12 in a natural pelvis 14 in connection with the implantation of a hip prosthesis at an implant site 16. The acetabulum has an acetabular centre 18 through which passes an acetabular axis 20, and the acetabular cup 10 has a polar axis 30 passing through the centre of rotation 32 of the acetabular cup, which in the case shown in Figure 1A is coincident with the centre of rotation 18 of the acetabulum when the cup is installed (however the cup centre need not necessarily be coincident with the acetabular centre). The acetabular cup has a face 34.

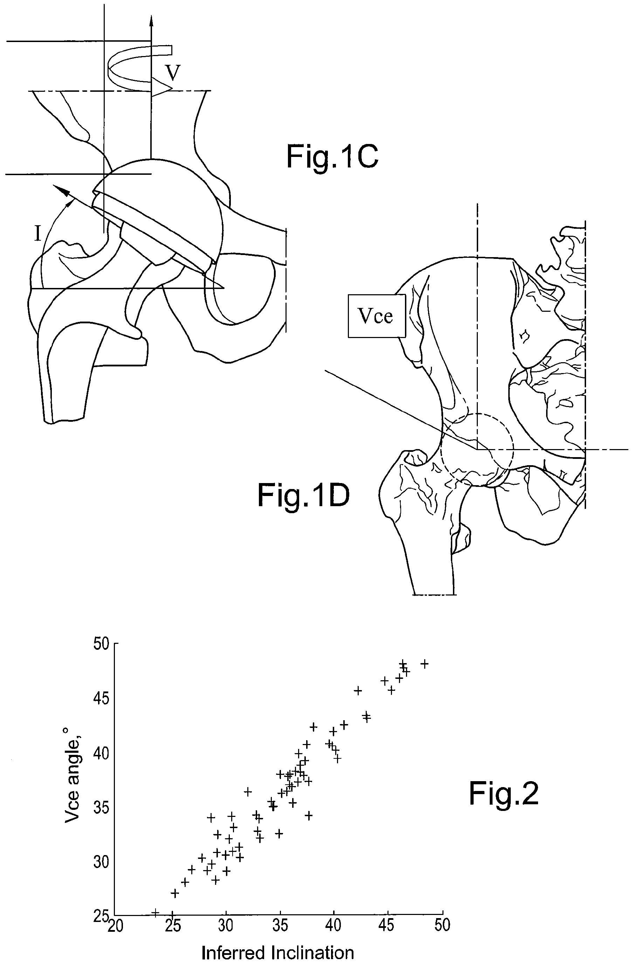

Cup orientation and position are defined in terms of inclination angle (angle from the transverse plane) and version angle (angle from the coronal plane), and cup centre. Figure 1C is a diagrammatic view of an acetabular cup installed in a pelvis indicating how orientation of the cup is defined by inclination angle I and version angle V. There are three distinct definitions of inclination and version, depending on the assessment used to determine the angle: operative, anatomical and radiographic, as explained by Murray (J Bone Joint Surg Br. 1993 Mar;75(2):228-32). Conversion of inclination or version angle from one definition to another is well understood can be done mathematically. Throughout the specification the terms inclination and version may be used to refer to angles according to any definition, with the knowledge that conversion between the various definitions is well known in the art. Throughout the specification the terms inclination and version may be used to refer to inclination and version of acetabular components or any other component of a device, e.g. a pin or cup guide etc.

Embodiments of devices according to the present inventions were devised by studying the relationship between local acetabular landmarks and optimal acetabular cup placement. Bone morphology analysis was carried out using specially developed scripts to analyse the repeatability of landmark relationships between people. The basic process of initial investigation was as follows:
a) segment patient data from CT (computed tomography) scans for a plurality of patients
b) convert segmented scan into cloud of points
c) use a script to locate the acetabulum and acetabular rim profile
d) use a script to define with geometrical shapes such as spheres, a range of local acetabular bony landmarks
e) use a script to relate the landmark locations and rim profiles between patients
f) use a script to measure a range of standard surgical acetabular morphological measurements, such as centre edge angle, which can be measured from 2D radiographs
g) look at repeatability and correlations within this data
h) use a script to assess locating techniques onto the acetabulum which could be replicated mechanically

Centre edge angle (Vce, also known as 'coverage angle') is a standard surgical acetabular morphological measurement that can be measured from 2D radiographs. Figure 1D shows a hip joint with the centre edge angle, Vce, of the acetabulum indicated.

Analysis was carried out on data from 72 patients to investigate the correlation between Vce angle and inclination by measuring standard two-dimensional Vce angle from the 72 patients and comparing it to the inferred inclination measured as the angle between a vector, defined between a point on the superior rim and the acetabular centre, and the transverse plane. The correlation between Vce angle and inferred inclination is shown in Figure 2. The correlation coefficient is 0.968. The data analysis demonstrated a strong correlation between 2D measured centre edge angle and cup inclination angle.

Next, the locations of repeatable bony landmarks from which the optimal cup position could be defined were determined using the steps set out below. In this process, the repeatable bony landmarks were referenced from the bony prominence in the obturator externus tendon groove, which is a useful landmark that can be easily visually identified by the surgeon during hip replacement.
1. Collect sample of pelvic CT data, selecting either the left or right hemi pelvis to focus on. Segment CT data, taking care to remove osteophytes..
2. Locate the rim of the acetabulum (not including the fovea) with at least 60 points. This can be done manually or with an automated script.
3. Fit a sphere to the acetabulum and locate the acetabular centre. This can be done manually or with an automated script.
4. Orientate the pelvis in the coronal plane by aligning the two anterior superior iliac spines (ASISs) along an axis.
4. Measure the coverage angle by projecting the acetabular rim profile into a 2D image in the coronal plane. Generate two vectors. Vector 1 is between the most overhanging superior point on the acetabulum and the acetabular centre. Vector 2 is perpendicular to the axis between the two ASISs. The coverage angle is the acute angle between these two vectors.
5. Orientate all pelvises into the anterior pelvic plane (APP) (plane through the right and left ASISs and the mid-point of the pubic tubercles). Define XY as the transverse plane, XZ as the coronal plane and YZ as the sagittal plane.
6. Extract all of the acetabular rim profiles with their acetabular centres, in the APP. Exclude acetabular rim profiles with severe dysplasia (defined as a coverage angle less than 25 degrees) or severe pincer impingement (defined as a coverage angle over 50 degrees).
7. Define an axis through the centre of the acetabulum, parallel to the X axis.
8. Project the rim profile into the sagittal plane. This should look like a distorted incomplete circle.
9. Locate the bony prominence in the obturator externus tendon groove. Also project this point into the 2D plane.
10. Moving clockwise from the bony prominence pick a rim profile point every 5 degrees (excluding points where the circle is broken, at the fovea) around the acetabular rim.
11. For each of the points in 5 degree intervals, calculate the angle subtended between a vector between the point and the acetabular centre, and a vector between the centre and the bony prominence. Project this angle into a plane perpendicular to a best fit plane through the rim points, and the transverse plane. This angle is related to inclination, and can be referred to as inferred inclination.
12. For each of the points in 5 degree intervals, also calculate the angle related to version. Couple the points with their diametrically opposite point (e.g. 25 and 205 degrees). Calculate the angle between a vector between the two points, and the Y axis. This angle is related to version, and can be referred to as inferred version.
13. First focussing on inclination. For each of the 5 degree interval rim points, define the correlation between the centre edge angle and the inferred inclination across all of the acetabulums.
14. Select the rim point angular position with the highest correlation to coverage angle. An angular definition for a point on the rim which is highly correlated to the coverage angle has now been obtained.
15. Second, focussing on version. For each of the 5 degree interval rim points, define the correlation between the vector between each rim point and its diametrically opposite couple, projected into the transverse plane, and the patient natural version. Also record the overall range of version across each subject at the defined 5 degree interval rim point.
16. Select three rim points. The superior rim point is the point, defined by its angular relationship with the bony prominence, which correlated highest with centre edge angle. The anterior point is chosen as the rim point which correlated highest with natural version, and also has an acceptably low range of version across all subjects. The posterior point is defined 180 degrees from the anterior point. Define a vector between the superior rim point and the acetabular centre. Rotate this vector about the Y axis by an amount calculated from the coverage angle. This amount is found by defining a best fit relationship between coverage angle and inferred inclination, and using this to rotate the vector to a target position 40 degrees from vertical. The cup normal is the cross product of this vector and a vector between the anterior and posterior rim points. The final cup centre sits at the midpoint of the anterior and posterior rim points.

This demonstrates a way of finding the rim points, which generate a cup plane from which the global orientation of the pelvis and therefore the optimal cup orientation and/or positioning can be referenced. Devices that use these rim points identified by the above method are described below.

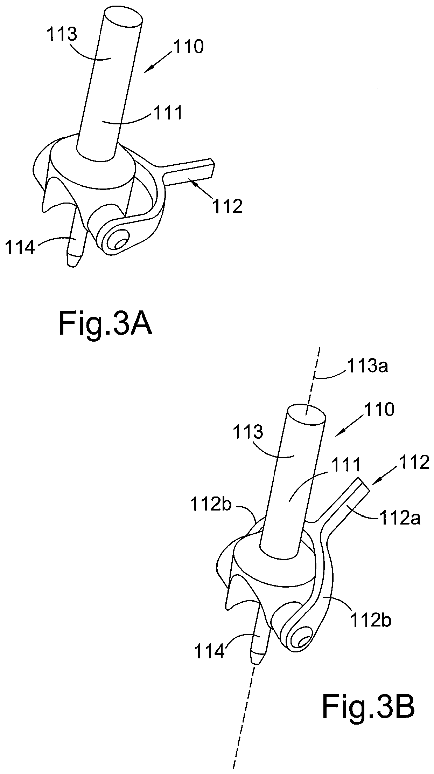

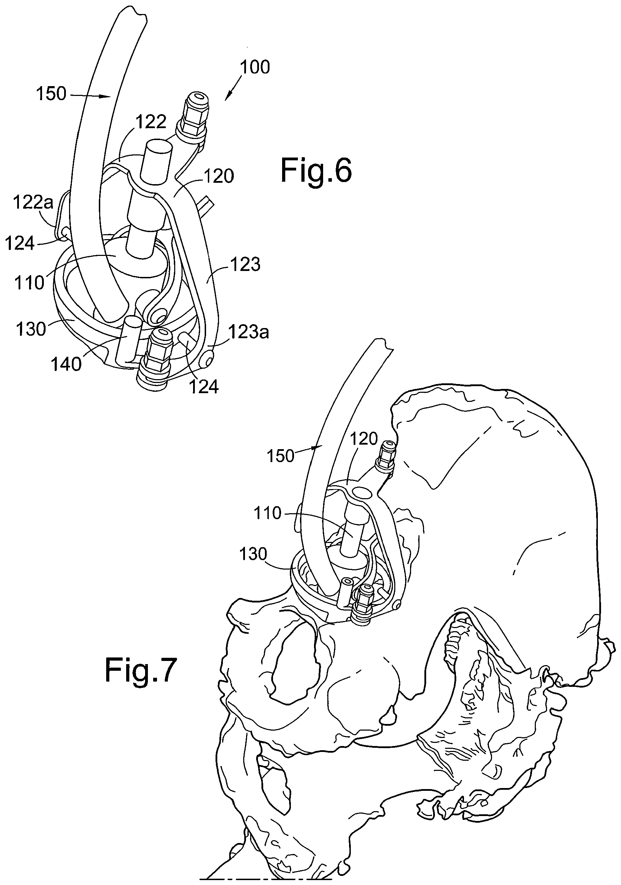

Referring to Figure 6, an assembled device 100 for facilitating alignment of an acetabular component relative to the pelvis of a subject, according to an embodiment of the present invention, is shown. The device 100 comprises a first portion 110 as shown in Figure 3B and a second portion 120 attachable to the first portion 110. The assembly further comprises a cup-shaped component 130 and introducer 150 attached to the device.

Referring to Figure 3B, the first portion 110 of the device 100 is shown. The first portion comprises a device body 111 having a distal shaft portion 113 and a proximal shaft portion 114, the distal and proximal shaft portions 113,114 being elongate, having longitudinal axes that are collinear along a longitudinal axis 113a. The first portion 110 also comprises a locator 112 for locating to a mounting location on the superior acetabular rim of a subject in use (the locator may be referred to as a superior locator 112). The superior locator 112 comprises a fork having fork ends 112b which attach to the device body 111 in use and a fork stem 112a for contacting a subject's acetabular rim when installed. The fork ends 112b attach to the device body 111 such that the superior locator 112 can pivot relative to the device body 111, about an axis perpendicular to the longitudinal axis 113a of the device body.

The superior locator 112 can be adjusted pivotally relative to the device body 111 to a position relative to the device body 111 selected by the user and locked in the selected configuration via locking means (not shown) such as a screw locking means.

The superior locator 112 is an inclination locator, in that adjustment of the superior locator 112 to a selected position allows the user to configure the device 100 such that an acetabular component can be aligned substantially to within a prescribed inclination angle range, as will be described in more detail later. As such, the first portion 110 of the device is the inclination reference portion of the device. There are preferably markings (not shown) on the device body 111 of the first portion 110 to indicate the angles, relative to the longitudinal axis 113a, that the superior locator 112 can be aligned to during adjustment.

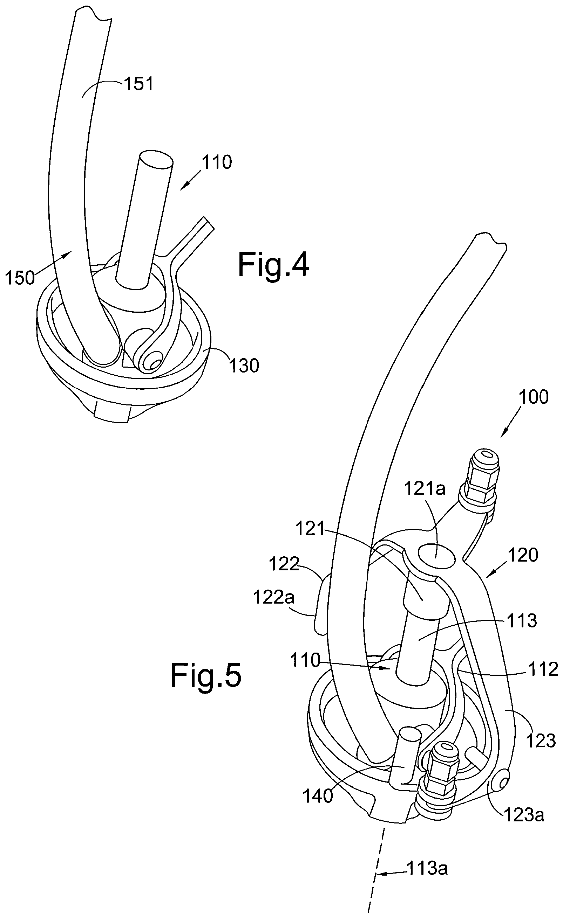

Referring to Figure 4, a first portion 110 of the device is shown attached to an introducer 150, for inserting an acetabular component into a subject's acetabulum. The introducer 150 has a handle 151, to be grasped by the user, and cup-shaped component 130 attached at its proximal end. For the introducer 150 shown in the present embodiment, the proximal shaft 114 of the first portion 110 of the device inserts in a corresponding bore (not visible in the figures) of the introducer 150, however the device 100 can have suitable attachment means for attaching to other pre-existing introducers. The device 100 has suitable means for rigidly securing the first portion 110 relative to the introducer 150; for example, the proximal shaft 114 may be externally threaded, the corresponding bore of the introducer 150 being correspondingly internally threaded to secure the first portion 110 to the introducer 150.

The cup-shaped component 130 at the proximal end of the introducer may be interchanged with cup-shaped components of different diameters. The assembly can be provided as a kit, in which a range of cup-shaped components of differing diameters are provided.

Referring to Figure 5, the second portion 120 of the device is shown being assembled to the first portion 110. The second portion 120 comprises a body portion 121 having a central bore 121a adapted to receive the distal shaft portion 113 of the first portion 110, such that the second portion 120 may slide over and can be received on the distal shaft portion 113 of the first portion 110. The first portion 110 and second portion 120 each include corresponding means for keying the first and second portions 110, 120, by locking them together in an axial plane when assembled, such that the second portion cannot rotate relative to the first portion about the longitudinal axis 113a. When the first portion 110 and second portion 120 of the device are assembled, the respective device bodies 111, 121 together form a device body of the assembled device, having a longitudinal axis 113a.

Extending from the body 121 of the second portion 120 are a first locator 122 for locating to a mounting location on the anterior acetabular rim of a subject in use (referred to hereafter as the anterior locator 122) and a second locator 123 for locating to a mounting location on the posterior acetabular rim of a subject in use (referred to hereafter as the posterior locator 123). The anterior and posterior locators 122, 123 are arms that extend away in opposite directions from the body portion 121 of the second portion 120. The anterior and posterior locators 122, 123 are curved arms, each curving away from its attachment point with the body portion 121, such that the pair of locators 122, 123 substantially form a C-shape. The distal end 122a of the anterior locator is adapted to locate to a mounting location on the acetabular rim when installed and the distal end 123a of the posterior locator is adapted to locate to a mounting location on the posterior rim when installed.

The second portion 120 of the device 100 is a version reference portion, allowing alignment of an acetabular component to a prescribed version, as will be described in more detail later.

Referring to Figure 6, in the present embodiment, the anterior and posterior locators 122, 123 are resilient and the distal end 122a, 123b of each of the anterior and posterior locators 122, 123 can be adjustably secured to the cup-shaped component 130 in such a way to selectively adjust the spacing between the distal ends 122a, 123a of the anterior and posterior locators. Each of the distal ends 122a, 123a has an aperture for receiving an externally threaded screw 124. Each screw is receivable in a corresponding internally threaded bore (not visible in the figures) in the cup-shaped component 130. As each screw 124 is screwed into or out of the cup-shaped component 130, this allows the user to adjust the spacing between the distal ends 122a, 123a of the anterior and posterior locators 122, 123 to substantially match the diameter of a subject's acetabular rim that the device is to be installed in. In this way, the device 100 can be adjusted to suit subjects of differing acetabular rim diameter.

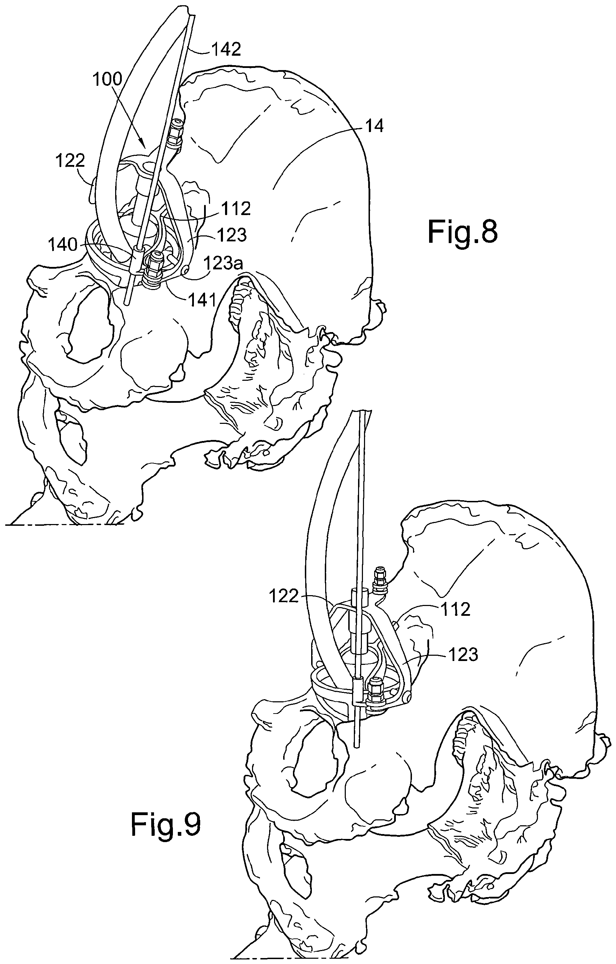

Referring to Figure 8, the second portion 120 further comprises an alignment reference element 140, being an alignment pin placement guide. The alignment reference element can be aligned with an identifiable natural landmark of the subject, in order to orient the device 100 to the subject in the plane of the acetabular rim for installation and therefore locate the locators 112, 122, 123 to their respective mounting locations on the acetabular rim, as will be described in more detail later. The alignment pin placement guide 140 is rigidly attached to the distal end 123a of the posterior locator by an extension portion 141 which extends from the distal end 123a of the posterior locator, in a plane substantially perpendicular to the longitudinal axis 113a of the device 100. The alignment pin placement guide 140 has a bore for receiving a device alignment pin 142 therethrough, for attachment to a subject's pelvis, as shown in Figure 8. The device alignment pin 142 can be a standard bone pin.

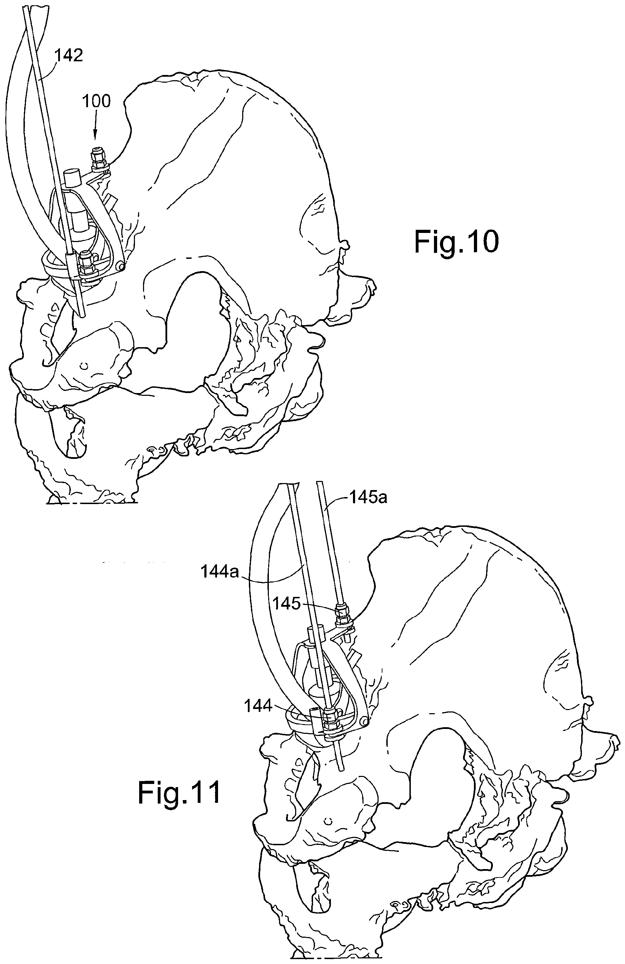

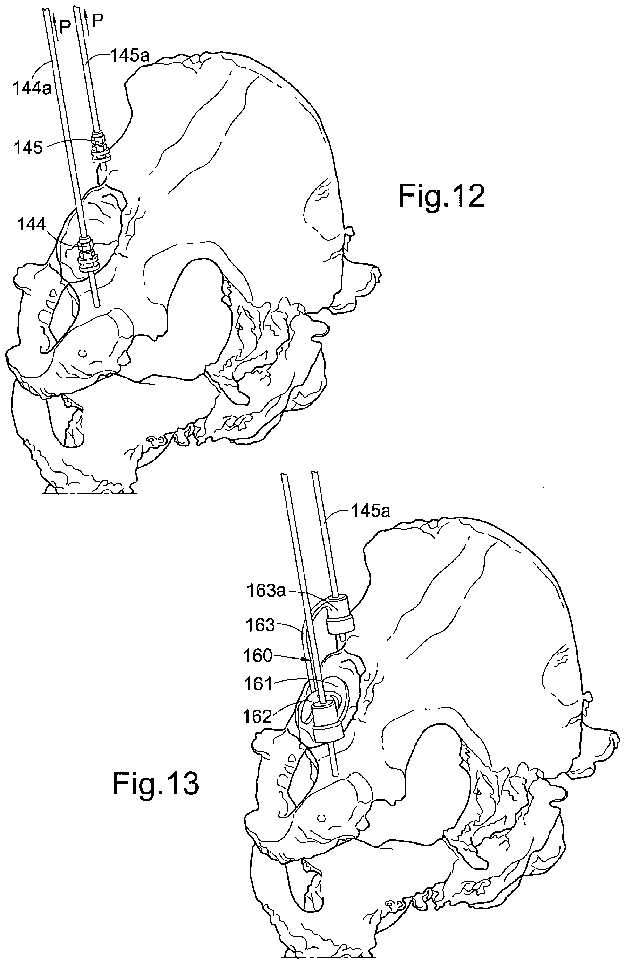

Referring to Figure 11, the second portion 120 further comprises a further pair of pin placement guides 144, 145. One of the pin placement guides 144, is rigidly attached to the extension portion 141 of the posterior locator 123. The other of the pin placement guides 145 is rigidly attached to an extension portion 146 extending from near the central bore 121a of the device body 121 of the second portion. Each of the pin placement guides 144, 145 has a bore for receiving an acetabular component alignment pin 144a, 145a therethrough, for attachment to a subject's pelvis, as shown in Figure 11. The pins 144a, 145a, can be standard bone pins. Unlike the alignment pin placement guide 140, the pin placement guides 144, 145 comprise cable glands or other suitable collets that can be tightened onto the respective pins 144a, 145a such that once the cable glands are tightened, the cable glands cannot pivot relative to the respective bone pin therein. Referring to Figure 12, the cable glands 144, 145 are releasably secureable to the device 100, such that the device can be removed from the cable glands 144, 145, leaving pins 144a, 145a secured in place in a subject's pelvis at a prescribed orientation, and each of the cable glands 144, 145 at a prescribed spacing away from the subject's pelvis. Once installed using the device, the pins 144a, 145a are oriented parallel with an axis P, which is parallel with a prescribed orientation for the acetabular component polar axis once installed.

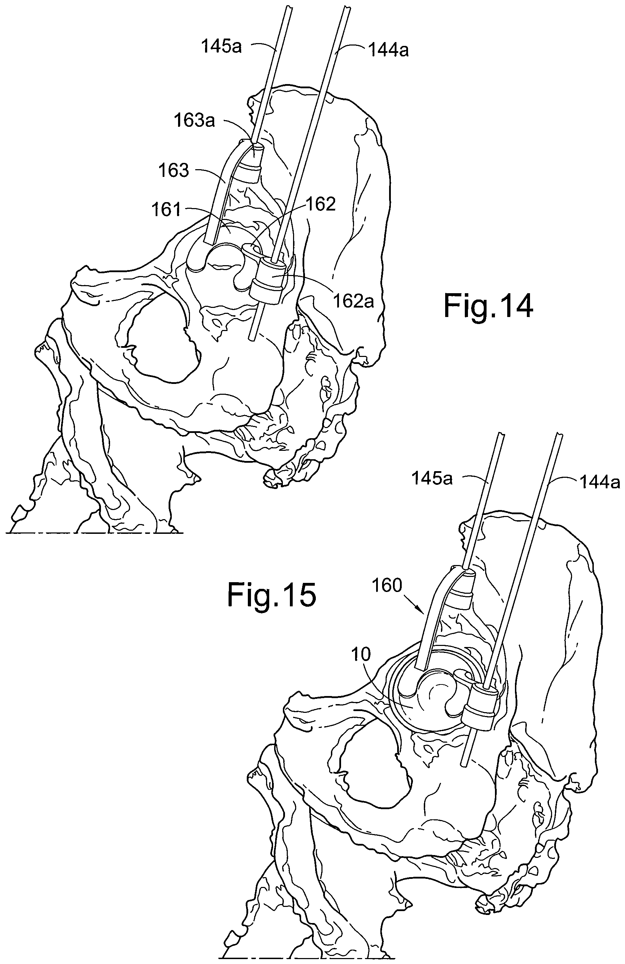

The installed acetabular component alignment pins 144a, 145a can be used for supporting a secondary guide, such as a cup guide 160 as shown installed on the pins in Figures 13 and 14, for assisting alignment of an acetabular component. The cup guide 160 has an arcuate flange portion 161. Extending from the arcuate flange portion 161 are first and second extender elements 162, 163. At the distal end of each extender element 162, 163 is a hood 162a, 163a, each having a bore sized to receive a respective pin 144a, 145a. The first hood 162a can be placed over the first pin 144a and the second hood 163a can be placed over the second pin 145a until the hoods 162a, 163a bottom out on the corresponding cable glands 144, 145. The cup guide 160 is designed such that when the hoods 162a, 163a are installed over the corresponding cable glands 144, 145, the arcuate flange portion 161 of the cup guide is oriented and positioned to be parallel and adjacent with the rim of the acetabular component when the acetabular component is installed in the prescribed orientation and position. In this way, the user can easily orient and position an acetabular component in the acetabulum at the prescribed orientation and position. The cup guide 160 can be provided with the device 100 as part of a kit.

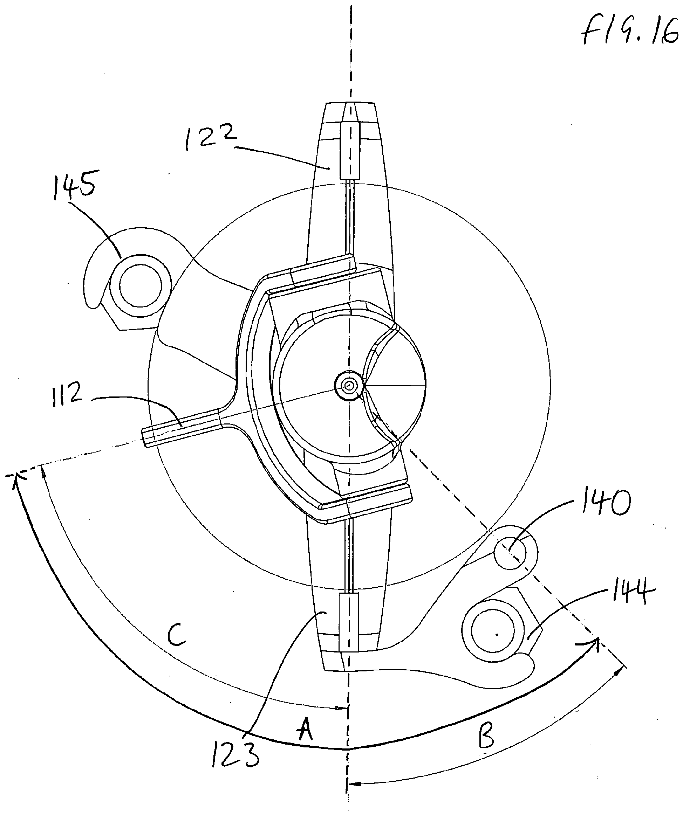

Referring to figure 16, when the first and second portions 110, 120 are assembled, the angle A between the alignment pin placement guide 140 and the superior locator 112, as measured about the longitudinal axis 113a of the device is 122°. The angle B between the alignment pin placement guide 140 and the posterior locator 123, as measured about the longitudinal axis 113a of the device is 46°. The anterior locator 122 is arranged at 180° from the posterior locator 123, as measured about the longitudinal axis 113a of the device is 122°. By means of the specific angular arrangement of the superior, anterior, and posterior locators 112, 122, 123, the locators will locate to respective mounting locations from which optimal cup orientation and/or position can be defined when the alignment pin placement guide 140 is aligned with the chosen natural landmark. The angles A and B may each be increased / decreased by 10° or so and still allow for cup placement within a safe zone of inclination and to an anatomical version. In other words, the angular arrangement of each of the locators 112, 122, 123 relative to the alignment reference element 140 may be varied by + 10° from that shown in Figure 16, and still allow for cup placement within a safe zone of inclination and to an anatomical version.

In operation, in order to install the device such that an acetabular component can be oriented and positioned to a prescribed orientation and position relative to a subject, firstly the subject's centre edge angle and femoral head diameter for the hip joint at which the device is to be installed are measured from a planar radiograph (this information could of course be measured directly from the subject's implant site, or from CT data). The device 100 is configured to orient and position the pin placement guides 144, 145 to a prescribed orientation and position relative to the subject when the device 100 is in a seated position in the subject's acetabulum, so that the prescribed orientation and position can be used as a reference for aligning an acetabular component (i.e. the acetabular component's polar axis can be aligned to be parallel with the axis that the pin placement guides 144, 145 are oriented to by the device 100 when in its seated position, by means of aligning the acetabular component to pins or a cup guide installed using the pin placement guides 144, 145; the acetabular component can be aligned to a particular position based on the position that pins or a cup guide are installed at using the device 100).

Referring to Figures 3A and 3B, the superior locator 112 is adjusted relative to the device body 111 to control the inclination that the longitudinal axis 113a of the device will be oriented to when in a fully seated position in the subject. The markings (not shown) on the device body 111 are each assigned a centre edge angle value, such that when the superior locator 112 is aligned with the marking corresponding to the centre edge angle value for the subject's acetabulum at which the device is to be installed, the superior locator 112 will be positioned relative to the device body 111 such that the resulting orientation of the pin placement guides 144, 145 when the device is in its seated position in the subject should be within a predetermined safe zone of inclination angle relative to the subject (i.e. the acetabular component, when aligned to the orientation output by the pin placement guides 144, 145, should have an inclination angle within a predetermined safe zone). The device is preferably designed such that the prescribed safe zone of inclination is 35 to 45°, however it will be understood that the device can be manufactured to align to other target inclination angle ranges.

The user does not have to carry out any calculations using the centre edge angle value measured from the subject to install the device to within the prescribed inclination angle range; instead the device is calibrated such that when the superior locator 112 is adjusted such that there is alignment with the marking corresponding to the measured centre edge angle value, the superior locator 112 is tilted by the appropriate amount relative to the device body in order to orient the device to within the prescribed inclination range when installed.

A cup-shaped component 130 of appropriate size is selected from a range of cup-shaped components of differing diameters. The cup-shaped component 130 is selected to have a diameter that matches, or most closely matches out of the range of cup-shaped components available, the subject's femoral head diameter, as measured from the subject's radiograph for example. In this way, the cup-shaped component can be seated in the subject's acetabulum during installation of the device 100, to define the acetabular centre of the subject. The selected cup-shaped component 130 is attached to the proximal end of the introducer 150 and the first portion 110 is inserted in the introducer 150 to assemble the first portion 110 to the introducer 150, as shown in Figure 4. The cup-shaped component 130 defines the acetabular centre of the subject when the cup-shaped component 130 is seated to rest in the acetabulum. The superior locator and acetabular centre as defined by the cup-shaped component 130 define a reference line relative to the subject when the device is installed.

Referring to Figures 5 and 6, the second portion 120 of the device is assembled to the first portion 110 by sliding the second portion 120 over the distal shaft portion 113. If needed, the spacing between the distal ends 122a, 123a of the anterior and posterior locators 122, 123 can be adjusted depending on the subject's acetabular rim diameter / femoral head diameter.

Referring to Figure 7, the introducer 150 is used to approach the assembled device 100 to the unreamed acetabulum of the subject medially (vertically in space). The device 100 is designed for installing in a subject when the subject is in a lateral decubitus position. The device 100 shown in Figures 3A to 16 is for guiding alignment of an acetabular component in a left hip, however it will be understood that a suitable device can be manufactured for guiding alignment of an acetabular component in a right hip, the device for right hips being a mirror image of the device for left hips. In other words, the superior locator will be located anticlockwise from the anterior locator in a device for the left hip whereas the superior locator will be located clockwise from the anterior locator in a device for the right hip, as viewed from the underside of the device. For installing the device 100 at the left hip of a subject, the subject should be lying in the right lateral decubitus position.

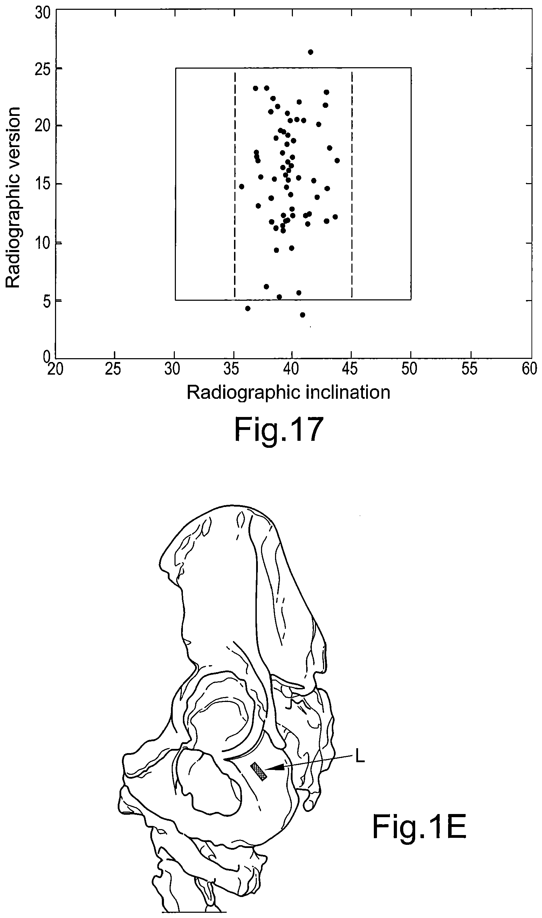

Referring to Figure 8, a device alignment pin 142 is inserted through the alignment pin placement guide 140. The surgeon should identify the subject's obturator externus tendon groove, a bony prominence that is easily identifiable as a natural landmark of any subject. This landmark could also be defined as the ridge in the ischial bone, but will be referred to hereinafter as the obturator externus tendon groove or 'the natural landmark'. Referring to Figure 1E, this natural landmark is identified by the mark labelled L. The device 100 is rotated within the acetabulum until the alignment pin placement guide 140 aligns with this predetermined natural landmark. When the alignment pin placement guide 140 is lying over the natural landmark, the device alignment pin 142 is located to rest on the natural landmark or is inserted into the subject's bone at that point.

Referring to Figures 8 and 9, with the device alignment pin 142 resting on the natural landmark, the introducer 150 is then tilted until the superior 112, anterior 122 and posterior 123 locators rest on the subject's acetabular rim. In embodiments where the device alignment pin 142 is intended to be inserted in the subject's bone, the alignment pin placement guide 140 is designed such that when a device alignment pin 142 has been inserted in the subject's pelvis, the alignment pin placement guide 140 and therefore the device 100 can pivot relative to the device alignment pin 142. The second portion 120 can slide relative to the first portion 110 to allow the superior, anterior and posterior locators 112, 122, 123 to each rest on the acetabular rim. By aligning the alignment pin placement guide 140 to the natural landmark, the superior locator 112 can automatically be located to rest on a predetermined mounting location on the superior acetabular rim of a subject in use, due to the fixed angular arrangement of the alignment pin placement guide 140 and superior locator 112 in the assembled device. The mounting location on the superior acetabular rim is the point which was found to correlate highest with centre edge angle, as determined from the data analysis for sample patients discussed above. Similarly, the anterior locator 122 locates to a mounting location on the anterior rim which, when combined with the posterior rim point, produces a vector which correlated highest with natural version, and also had an acceptably low range of version across all subjects. The posterior locator 123 locates to a mounting location on the posterior rim which is 180° from the anterior mounting location.

The superior, anterior and posterior mounting locations on the acetabular rim would be hard for the surgeon to identify reliably either visually or by palpation, however the device 100 allows the user to locate the superior, anterior and posterior locators 112, 122, 123 to their respective mounting locations via alignment of the alignment pin placement guide 140 with the natural landmark.

Once the superior, anterior and posterior locators 112, 122, 123 are each resting on the acetabular rim, and the cup-shaped component 130 is seated in the acetabulum, the device 100 is in its fully seated position. The first and second acetabular component alignment pins 144a, 145a are then inserted through the corresponding cable glands 144, 145. One of the pins 144a is drilled into the subject's ischium and the other of the pins 145a is drilled into the subject's ilium. It will be understood that the location that the cable glands 144, 145 could be arranged relative to the device body to locate the acetabular component alignment pins 144a, 145a to other suitable locations than as shown in Figure 11. The cable glands 144, 145 are tightened around the corresponding pins inserted therethrough. The device alignment pin 142 can be removed before or after the acetabular component alignment pins 144a, 145a are installed in the subject.

Referring to Figure 12, the device 100 is twisted and removed from the cable glands 144, 145, leaving the acetabular component alignment pins 144a, 145a, with cable glands 144, 145 attached thereto, installed in the subject. The acetabular component alignment pins 144a, 145a that have been installed using the device 100 are aligned at a prescribed orientation and position relative to the subject, as defined by the superior, anterior and posterior locators having been placed on the superior, anterior and posterior mounting locations. The superior, anterior and posterior locators define a plane relative to the subject when the device installed, which is then used to define a prescribed plane that a cup face of an acetabular component should be aligned to. The prescribed orientation that the pins 144a, 145a are aligned to by the device 100 is an orientation that the acetabular component to be installed is to be aligned to, and this prescribed orientation has a prescribed version and inclination. The prescribed orientation comprises a subject-specific version angle, based on the anterior and posterior mounting locations, so that the acetabular component can be aligned to an anatomical version for the particular subject. The prescribed orientation comprises (for the majority of subjects) an inclination angle within the range 35 to 45°. This prescribed orientation is controlled by the tilt angle of the superior locator 112 relative to the device body, which controls the amount at which the longitudinal axis 113a of the device tilts relative to the superior mounting location in a plane containing the superior mounting location and the acetabular centre (as defined by the cup-shaped component 130).

The prescribed position that the pins 144a, 145a are positioned to by the device 100 is governed by the mid-point between the anterior and posterior locators 122, 123 (i.e. the points of the device which contact the anterior and posterior acetabular rim respectively). Because the second portion 120 slides relative to the first portion 110 of the device, the second portion 120 also governs the depth that the cable glands 144, 145 are positioned at relative to the pelvis when installed. In this way, the second portion, including the anterior and posterior locators 122, 123, governs positioning of the pins 144a, 145a, such that the notional centre of the arcuate flange portion 161 of the cup guide 160 will be positioned at the prescribed point to which the centre of the acetabular component should be aligned when aligning the acetabular component in the prescribed position. The device does not aim to restore the natural acetabular centre position of the subject, as depending on the shallowness of the acetabulum, the acetabular cup centre may not be coincident with the natural acetabular centre. The device aims to provide a final cup centre position at a depth in the acetabulum whereby the rim of the acetabular cup implant is not proud and will therefore not impinge on any soft tissue. The three points that define this hip centre are then used to define the plane of the cup face, such that the cable glands 144, 145 are positioned at a prescribed height relative to the subject's native acetabulum. By means of the cup guide 160 positioned over the pins 144a, 145a with cable glands 144, 145 thereon, this allows an acetabular component to be positioned to a prescribed position relative to the subject's acetabulum, as well as to a prescribed orientation.

Referring to Figure 13, the hoods 162a, 163a of a cup guide 160 are slid over the acetabular component alignment pins 144a, 145a until the hoods bottom out on the cable glands 144, 145. When in this position, the arcuate flange portion 161 of the cup guide is oriented and positioned at a prescribed orientation and position, so that an acetabular component, such as a cup or reamer can be aligned to the arcuate flange portion 161 to orient and position it in the prescribed orientation and position relative to the subject. The cup guide 160 is designed such that when the hoods 162a, 163a are installed over the corresponding cable glands 144, 145, the arcuate flange portion 161 of the cup guide is oriented and positioned to be parallel and adjacent with the rim of the acetabular component when the acetabular component is installed in the prescribed orientation and position. In this way, the user can easily orient and position an acetabular component in the acetabulum at the prescribed orientation and position. The cup guide 160 can be provided with the device 100 as part of a kit.

In operation, in order to install an acetabular cup to the prescribed orientation and position, the acetabulum is then reamed. The cup guide 160 and/or acetabular component alignment pins 144a, 145a can be used to orient and/or position the reamers during sequential reaming to the necessary size and depth.

Once the acetabulum has been reamed, referring to Figure 15, an acetabular cup 10 is installed and impacted into the acetabulum. Any suitable cup introducer may be used to introduce the acetabular cup into the acetabulum. The cup guide 160 and/or acetabular component alignment pins 144a, 145a can be used to orient and/or position the acetabular cup during installation and/or impaction such that the acetabular cup is located at a prescribed orientation and position relative to the subject. Reaming, trialling cup size, and impacting can all be carried out beneath the installed cup guide 160 or alternatively, the cup guide 160 can be moved up the pins 144a, 145a or completely removed. If the cup guide 160 is removed during impaction, it can be replaced for use as a mid-/post-impaction check. The device 100 therefore facilitates alignment of an acetabular component, such as a reamer, trial cup or acetabular cup implant by defining a reference orientation and position to which the acetabular component can be aligned.

It will be understood that a natural landmark other than the obturator externus tendon groove may be used as the reference to align the alignment reference element 140 to. If the device is designed to use a natural landmark other than the obturator externus tendon groove, then the angles between each of the superior, anterior and posterior locators 112, 122, 123 and the alignment reference element, as measured about the longitudinal axis 113a of the device will differ from those described for device 100; however, the angles between each of the superior, anterior and posterior locators 112, 122, 123 will be as described above, so that the superior, anterior and posterior locators can each locate to their corresponding mounting location on a subject's acetabulum.

Theoretical analysis on CT-data from 65 subjects was carried out to determine theoretically at what orientation the device 100 would locate an acetabular cup to for each subject, and the results are shown in Figure 17. The data shows that for each subject, the device would position the cup to have radiographic inclination angle within the target range of 35 to 45°. The spread of radiographic version angle achieved for the subjects was greater, since the device orients a cup to align with anatomical version for each particular subject.

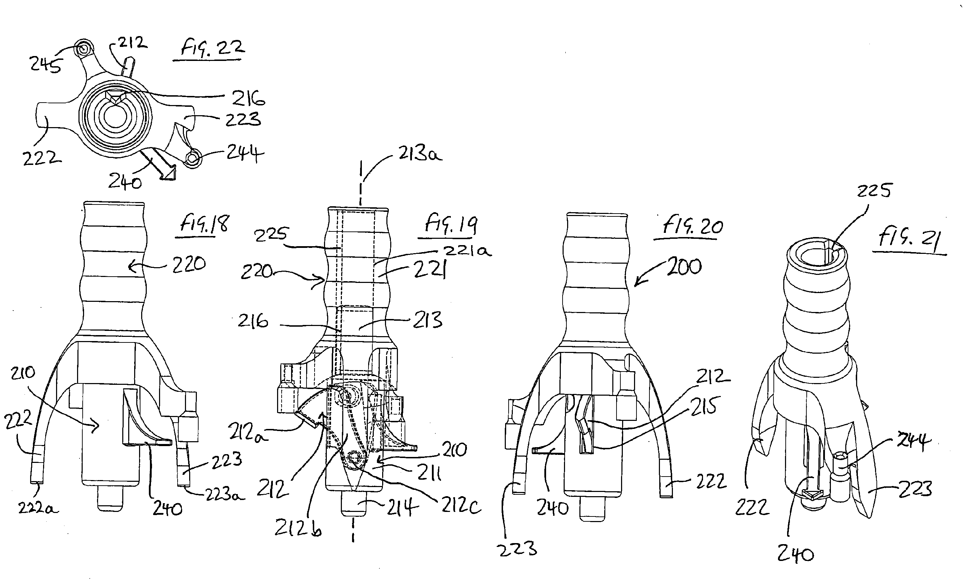

Referring to Figures 18 to 23, a further embodiment according to the invention is shown. Referring to Figure 20, an assembled device 200 is shown for facilitating alignment of an acetabular component relative to the pelvis of a subject. Device 200 is for use at the left hemipelvis; a device 200 for the right side would be a mirror image of the device in Figures 18 to 23. The device 200 is very similar to device 100 of Figures 3A to 16, however the device 200 has an alignment marker 240 for aligning with a predetermined identifiable landmark on the subject, rather than a pin placement guide 140 as on device 100. Similar elements of device 200 are denoted by similar reference numerals.

The device 200 comprises a first portion 210 and a second portion 220 mountable to the first portion 210, which are shown assembled together in Figures 18 to 23. Referring to Figure 19, the first portion 210 comprises a substantially cylindrical device body 211 having a distal shaft portion 213 and a proximal shaft portion 214, the distal and proximal shaft portions 213, 214, having longitudinal axes that are collinear along a longitudinal axis 213a. The first portion comprises a superior locator 212 movably attached to the device body 211, for locating to a mounting location on the superior acetabular rim of a subject. The superior locator 212 comprises a pivotable arm 212b that is pivotably movable relative to the device body 211 about an axis 212c perpendicular to the longitudinal axis of the device body 211. The superior locator 212 has a foot portion 212a at its distal end (remote from the pivotal connection 212c with the device body 211), for contacting a subject's acetabular rim when installed. The superior locator 212 is located within a hollow portion of the device body 211, with the foot portion 212a protruding through an aperture 215 in the device body 211 so that the foot portion 212a can contact a subject's acetabular rim.

The superior locator 212 can be adjusted pivotally relative to the device body 211 to a position relative to the device body 211 selected by the user and locked in the selected configuration via locking means (not shown) such as a screw locking means. There are preferably markings (not shown) on the superior locator 212 and/or the device body to indicate the angles relative to the longitudinal axis 213a that the superior locator 212 can be aligned to during adjustment.

Referring to Figures 19, 21 and 22, the second portion 220 of the device comprises a body portion 221 having a central bore 221a adapted to receive the distal shaft portion 112 of the first portion 110, such that the second portion 120 is slidable over and can be received on the distal shaft portion 213 of the first portion 210. The second portion 220 has a keyway 225 running longitudinally along the inner surface of the central bore 221a, adapted to receive a corresponding keying part 216 of the first portion 210, such that the second portion 220 cannot rotate relative to the first portion 210 about the longitudinal axis 213a.

Like device 100, the second portion 220 of the device 200 has an anterior locator 222 and a posterior locator 223, for locating to mounting locations on the anterior acetabular rim and posterior acetabular rim respectively. Unlike device 100, the anterior and posterior locators 222, 223 are not adjustable relative to the longitudinal axis 213a, but are rigid arms. Device 200 may be provided as a kit having a selection of second portions 220, each having a differing spacing between the distal ends 222a, 223a of the anterior and posterior locators 222, 223, to suit subjects of differing acetabular rim diameter.

Referring to Figure 21, instead of the pin placement guide of device 100, device 200 has an alignment marker 240 extending from the device body. The alignment marker 240 may extend from the first portion 210 or the second portion 220 of the device. The alignment marker 240 is rotationally fixed relative to the superior, anterior and posterior locators 212, 222, 223 in use, such that each locator is located at a fixed angle relative to the alignment marker 240 about the longitudinal axis 213a. As can be seen from Figure 21, the alignment marker 240 comprises an arrow shaped pointer that can be used to align with the identifiable natural landmark of the subject, in order to orient the device 200 to the subject, to locate distal ends of the locators 112, 122, 123 to rest on their respective mounting locations on the acetabular rim.

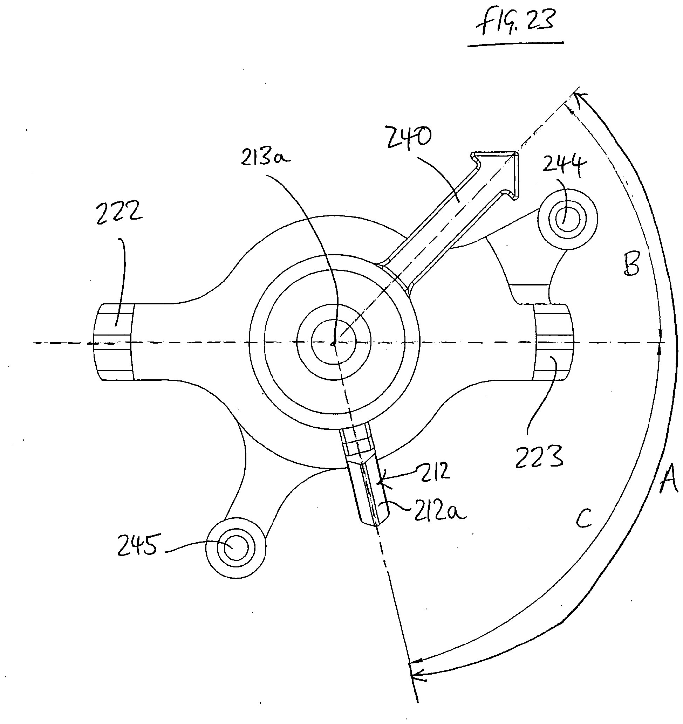

Referring to Figure 23, like device 100, device 200 has a pair of pin placement guides 244, 245, for receiving acetabular component alignment pins (not shown), for attachment to the subject's pelvis.

Referring to Figure 23, the angular arrangement of the superior locator 212, anterior locator 222, posterior locator 223, and alignment marker 240 of device 200 are the same as for device 100. As such, angle A between the alignment marker 240 and the superior locator 212 is 122°. The angle B between the alignment marker 240 and the posterior locator 223 is 46°. The anterior locator 222 is arranged at 180° from the posterior locator 223. These angles are measured about the longitudinal axis 213a of the device. The angles A and B may each be increased / decreased by 10° or so and still allow for cup placement within a safe zone of inclination and to an anatomical version. In other words, the angular arrangement of each of the locators 212, 222, 223 relative to the alignment reference element 240 may be varied by + 10° from that shown in Figure 23, and still allow for cup placement within a safe zone of inclination.

In operation, installation of the device 200 to a subject's acetabulum is very similar to that for device 100. Firstly the subject's centre edge angle and femoral head diameter for the hip joint at which the device is to be installed are measured from a planar radiograph. The superior locator 212 is then adjusted relative to the device 200 to control the inclination that the longitudinal axis 213a of the device will be oriented to when in a fully seated position in the subject. The markings (not shown) on the device are each assigned a centre edge angle value, so that the superior locator 212 can be adjusted until there is alignment with the marking assigned the centre edge angle value of the subject's acetabulum at which the device is to be installed. The user locks the superior locator 212 at the selected position. The tilt angle of the superior locator 212 relative to the device 200 controls the amount at which the longitudinal axis 213a of the device tilts relative to the superior mounting location that the superior locator 212 locates to. The device 200 is pre-calibrated to enable the longitudinal axis 213a of the device to be oriented to an inclination within a safe zone of 35 to 45° based on the centre edge angle measured from the subject, by aligning the superior locator 212 until there is alignment with the marking corresponding to the measured centre edge angle value.

Similarly with device 100, a cup-shaped component (not shown) of appropriate size is selected from a range of cup-shaped components of differing diameters based on the femoral head diameter measured from the subject. Alternatively a cup-shaped component having an diameter that can be varied in size can be used, the diameter being adjusted based on the femoral head diameter measured from the subject. The selected cup-shaped component is attached to the proximal shaft portion 214 of the device. The proximal shaft portion 214 is externally threaded (not shown), such that it can couple with corresponding internal threading of a cup-shaped component. Other means for attaching the cup-shaped component to the device may of course be used, such as press-fit attachment.

The device 200 with cup-shaped component assembled to it is then introduced to the unreamed acetabulum and the device 200 rotated within the acetabulum until the alignment indictor 240 aligns with the obturator externus tendon groove. When the alignment indictor 240 is aligned with the obturator externus tendon groove, the device 200 is then tilted until the superior, anterior and posterior locators 212, 222, 223 rest on the subject's acetabular rim. The second portion 120 can slide relative to the first portion 110 to allow the superior, anterior and posterior locators 212, 222, 223 to all rest on the acetabular rim. By aligning the alignment indictor 240 with the natural landmark, the locators can be automatically located to rest on predetermined mounting locations on the acetabular rim, which would otherwise be hard to identify reliably.

Once the superior, anterior and posterior locators 212, 222, 223 are resting on their corresponding mounting locations on the acetabular rim, first and second pins are inserted through the pin placement guides 244, 245 respectively. The pins are drilled into the subject's pelvis. The pin placement guides 244, 245 have elongate bores that align the pins parallel with the longitudinal axis 213a of the device when the pins are within the pin placement guides, such that the pins will be installed in the subject parallel with the longitudinal axis of the device.

The device 200 may then be removed from the pins, leaving the pins 200 installed in the subject. The pins are thus installed in the subject at an orientation that can be used as a reference orientation for aligning an acetabular component to, to align the acetabular component to the prescribed orientation defined by the reference plane defined by the superior, anterior and posterior mounting locations and the tilt of the superior locator 212 relative to the device 200.

Cable glands or collets (not shown) can be installed on each of the pins at a location defined using the location of the pin placement guides 244, 245 relative to the pins when the pins are installed in the pin placement guides. In this way, the cable glands can be positioned at a prescribed height relative to the subject's acetabular centre as defined by the cup-shaped component. The pins and cable glands can be used for supporting a cup guide, to be used as a reference for aligning an acetabular component such as a reamer, trial cup or acetabular cup implant to the prescribed orientation and position previously defined using device 200.

It will be understood that for both devices 100 and 200, the cup-shaped component to be attached to the proximal end of the device need not be cup shaped. Instead, any component that can be used to substantially define the subject's acetabular centre could be used, such as a tripod component, having three legs that seat within the acetabulum, to define the acetabular centre.

It will also be understood that instead of a cup-shaped component, the devices 100, 200, could be attachable to other acetabular components. For example, device 100 or 200 could be assembled directly to an acetabular reamer component, such that the device could be used to orient the reamer to a prescribed orientation and position relative to the acetabulum. In such a design, where the device is being used to orient and position an acetabular component directly within the acetabulum, there would be no need for alignment pins, such as pins 144a, 145a of the first embodiment, to act as reference guides for aligning the acetabular component after the device has been removed from the acetabulum.

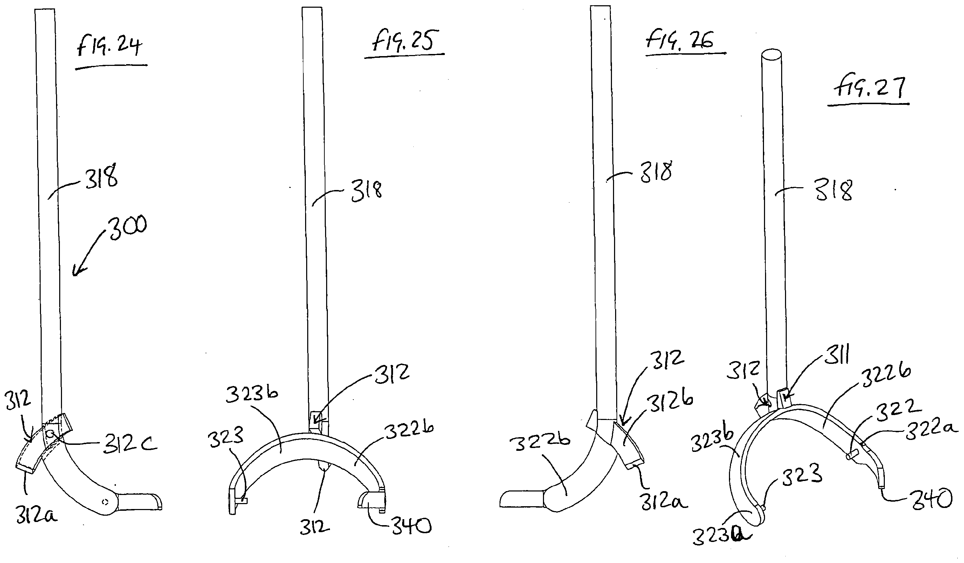

Referring to Figures 24 to 28, a further embodiment according to the invention is shown. Figures 24 to 28 show a further device 300 for facilitating alignment of an acetabular component relative to the pelvis of a subject. Device 300 is for use at the left hemipelvis; a device for the right side would be a mirror image of the device in Figures 24 to 28. Device 300 is similar to device 200 in that device 300 has an alignment marker 340 for aligning with a predetermined identifiable landmark on the subject and has superior, anterior and posterior locators 312, 322, 323 for mounting on an acetabular rim, the locators each being arranged at a fixed angle relative to the alignment marker 340 as measured about an axis of the device 313a. However, unlike devices 100, 200, the device 300 does not attach to an acetabular centering component such as a cup-shaped component 130, 230. Also, unlike devices 100, 200, the device 300 is not configured to orient reference pins to a prescribed orientation and position, which are then used as a reference for aligning an acetabular component after device 100, 200 has been removed; instead, device 300 has an elongate handle 318 which can be used as a reference for aligning an acetabular component, whilst the device 300 remains installed at the subject's acetabulum. Similar elements of device 300 are denoted by similar reference numerals to those used for devices 100, 200.

Unlike devices 100, 200, the device 300 is not comprised of first and second portions which are slidable relative to one another in use. Device 300 has a device body 311 and a superior locator 312 for locating to a mounting location on the superior acetabular rim. The superior locator 312 is a curved arm 312b that is slidably mounted to the device body 311. Referring to Figure 24, the superior locator arm 312b has a curved elongate slot (not shown) running along the arm. A pin 312c mounted in the device body 311 passes through the slot on the superior locator arm 312b, so that the arm 312b can slide relative to the device body 311. The superior locator 312 has a foot portion 312a at one end, for contacting a subject's acetabular rim when installed. As the superior locator 312 is adjusted by sliding relative to the device body 311, it effectively pivots about an imaginary pivot axis.

Like devices 100, 200, superior locator 312 of device 300 can be position adjusted relative to the device body 311 by the user and locked in a selected configuration via suitable locking means (not shown), such as a screw locking means. There are preferably markings (not shown) on the superior locator 312 and/or device body 311, each marking corresponding with a centre edge angle value, so that the superior locator 312 can be adjusted to align with the centre edge angle value measured for the subject's acetabulum at which the device is to be installed.

Referring to Figure 27, device 300 has an anterior locator 322 and a posterior locator 323, for locating to a mounting location on the anterior acetabular rim and posterior acetabular rim respectively. Unlike device 100, the spacing between anterior and posterior locators 322, 323 is not adjustable. A kit may be provided, comprising a plurality of devices 300, each having differing spacing between the anterior and posterior locators 322, 323, to suit subject's of differing acetabular diameter. The device 300 has first and second curved arms 322b, 322b that extend from the device body 311, forming a C-shape. The anterior and posterior locators 322, 323 are protrusions that extend inwardly from the distal ends 322a, 323a of each of the arms 322b, 323b respectively. The protrusions 322, 323 are configured to rest on a subject's acetabular rim when the device is installed.

Referring to Figure 27, the device 300 also comprises an alignment marker 340. The alignment marker 340 is the distal, pointed end of an extension of arm 322b, extending beyond the anterior locator 322. The alignment marker 340 can be used to align with the identifiable natural landmark of the subject, in order to orient the device 300 to the subject, to locate the locators 312, 322, 323 to rest on their respective mounting locations on the acetabular rim.

Referring to Figure 27, the device 300 comprises an elongate handle 318, extending from the device body 311. The elongate handle 318 can be grasped by the user to orient the device 300 to its seated position wherein each locator 312, 322, 323 rests on its corresponding mounting location. The elongate handle 318 also acts as a guide portion that an acetabular component can be aligned to, as the device 300 orients the elongate handle 318 to a prescribed orientation relative to the subject when the device 300 is installed in a seated position; therefore, at least part of the elongate handle can be used as a reference axis to which the polar axis of an acetabular component can be aligned parallel with, in order to align the acetabular component.

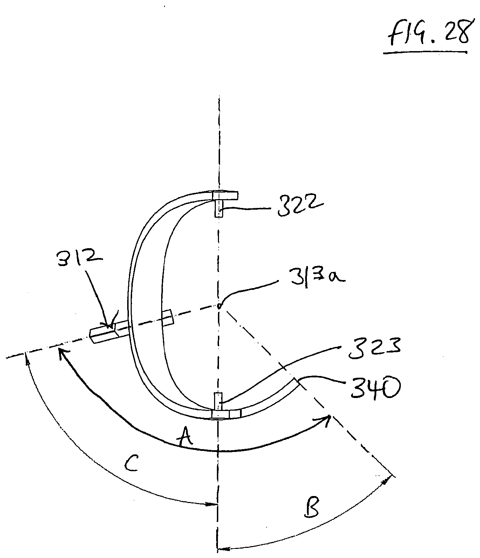

Referring to Figure 28, the angular arrangement of the superior locator 312, anterior locator 322, posterior locator 323, and alignment marker 340 of device 300 are the same as for devices 100 and 200. As such, angle A between the alignment marker 340 and the superior locator 312 is 122°. The angle B between the alignment marker 340 and the posterior locator 323 is 46°. The anterior locator 322 is arranged at 180° from the posterior locator 323. These angles are measured about an imaginary longitudinal axis 313a of the device, which passes through the mid point of the line between the anterior locator 322 and the posterior locator 323, and the vectors between the longitudinal axis 313a and each of the superior locator 312, anterior locator 322, posterior locator 323, and alignment marker 340, to define the angles having been projected on a plane perpendicular to the handle 318. The angles A and B may each be increased / decreased by 10° or so and still allow for cup placement within a safe zone of inclination. In other words, the angular arrangement of each of the locators 312, 322, 323 relative to the alignment reference element 340 may be varied by + 10° from that shown in Figure 28, and still allow for cup placement within a safe zone of inclination.

In operation, in order to install the device 300 to a subject's acetabulum such that an acetabular component can be oriented to a prescribed orientation relative to the subject, firstly the subject's centre edge angle and femoral head diameter for the hip joint at which the device is to be installed are measured from a planar radiograph. The superior locator 312 is adjusted relative to the device body 311 to control the inclination that the handle 318 will be oriented to when in a fully seated position in the subject. The markings (not shown) on the device are each assigned a centre edge angle value, so that the superior locator 312 can be adjusted until aligned with the marking assigned the centre edge angle value of the subject's acetabulum at which the device is to be installed. The user locks the superior locator 212 at the selected position. The tilt angle of the foot portion 312a of the superior locator 312 relative to the handle 318 controls the amount at which the handle 318 tilts relative to the superior mounting location that the superior locators 212 locates to when installed. The device 300 is pre-calibrated such that when the superior locator 312 is aligned with the marking that corresponds with the centre edge angle of the subject's acetabulum, the handle 318 will be tilted by an appropriate amount relative to the acetabulum when installed to orient the handle to within a prescribed inclination range.

The device is introduced to the implant site and the device is rotated relative to the acetabulum until the alignment marker 340 is aligned with the obturator externus tendon groove. When the alignment marker 340 is aligned with the obturator externus tendon groove, the device 300 is then tilted until the superior, anterior, and posterior locators 312, 322, 323 rest on the subject's acetabular rim. By aligning the alignment marker 340 with the predetermined natural landmark, the locators can be automatically located to rest on the corresponding predetermined mounting locations on the acetabular rim. When the device 300 is in its fully seated position, with each of the superior, anterior, and posterior locators 312, 322, 323 resting on the corresponding mounting locations, the handle 318 is thus aligned at a prescribed orientation relative to the subject that can be used as a reference orientation for aligning an acetabular component. For example, a reamer can be introduced to the acetabulum, the handle of the reamer being aligned parallel with at least part of the handle 318 of the device 300, in order to orient the reamer in the prescribed orientation. The C-shaped configuration formed by arms 322b, 323b of the device 300 partially encircles the acetabular rim, allowing an acetabular component to be introduced to the acetabulum whilst the device 300 remains in place in its seated position, so that the handle 318 can be used as a reference axis for orienting an acetabular component.

For device 300, the centre of the subject's acetabulum is inferred as the mid-point between the anterior and posterior locators 322, 323 when installed in the seated position. As device 300 is not used in combination with a cup-shaped component that attaches to the device, the device 300 only allows for alignment of an acetabular component to a prescribed orientation, not to a prescribed depth relative to the acetabulum.

The devices illustrated herein has many advantages, including the following:
- The devices take two pieces of patient specific information as inputs (centre edge angle and femoral head diameter) and auto-locate to find predetermined reference points on the rim when the device is placed in the acetabulum. The surgeon must make only one alignment to finalise the guide position. The two pieces of input information can be measured from a planar radiograph which is taken in all hip replacement procedures. The measured data are standard measurements which the surgeons will be well versed in taking.
- The devices offer a subject-specific guide in terms of cup version angle. Prior art basic guides assume an ideal version of 15°, whereas there is in fact much wider anatomical variation in version than inclination. The device aims for assisting alignment of an acetabular component to anatomical version and an optimal inclination.
- The devices can be used in situations of reduced visibility, such as during minimally invasive surgery, where it is not possible to adequately see all the cues required for visual alignment.
- The devices require no capital investment. It is therefore available to high and low-volume surgeons (one may argue that low-volume surgeons are those with a particular need for a guidance device owing to their reduce amount of experience).
- The devices can be designed to be disposable and the cost would be comparable to that of other surgical consumables.
- The devices do not have any lead time, such as waiting for analysis of CT data and manufacture of a patient matched device
- The invention can be used to assist in aligning an acetabular component to not only a prescribed orientation, but also a prescribed position. Devices according to the present invention can be used to indicate an acetabular component centre position to align to.

## Claims

1. A device (100, 200, 300) for use in facilitating alignment of an acetabular component relative to the pelvis of a subject, the device having a device body and at least a first locator (112, 212, 312) for placement on a first predetermined mounting location on the acetabular rim of a subject, a second locator (122, 222, 322) for placement on a second predetermined mounting location on the acetabular rim and a third locator (123, 223, 323) for placement on a third predetermined mounting location on the acetabular rim, the device defining an orientation and/or position with respect to said subject when installed for facilitating alignment of an acetabular component, **characterised in that** the device further comprises an alignment reference element (140, 240, 340) for aligning with a predetermined natural landmark of the subject in use, the natural landmark being identifiable by the user, the first, second and third locators being situated relative to the alignment reference element (140, 240, 340) such that upon alignment of the alignment reference element with the natural landmark, the locators locate to their respective mounting locations, wherein the natural landmark is a predetermined landmark which is other than on the acetabular rim.

2. A device according to claim 1, wherein said locator or one of said locators is a superior locator (112, 212, 312) for placement on a predetermined mounting location on the superior acetabular rim, one is an anterior locator (122, 222, 322) for placement on a predetermined mounting location on the anterior acetabular rim and one is a posterior locator (123, 223, 323) for placement on a predetermined mounting location on the posterior acetabular rim.

3. A device according to claim 1, wherein the alignment reference element comprises a pin placement guide (140) for guiding placement of a pin in the subject's pelvis at the natural landmark or it comprises an alignment marker (240, 340) that can be aligned with the natural landmark in use.

4. A device according to any preceding claim, wherein the device is configured such that the alignment reference element (140, 240, 340) is to be aligned with a bony prominence in the obturator externus tendon groove of a subject.

5. A device according to any of claims 2 to 4, wherein the spacing between the anterior (122) and posterior locators (123) is adjustable by the user.

6. A device according to any of claims 2 to 5, wherein the device body has first (110, 210) and second portions (120, 220), the superior locator (112, 212) being on or coupled to the first portion of the device body in use and the anterior and posterior locators being on or coupled to the second portion in use, the second portion being slidably mounted to the first portion in use.

7. A device according to any of claims 2, 5, or 6, wherein the anterior locator (122, 222, 322) is situated substantially diametrically opposite the posterior locator (123, 223, 323).

8. A device according to any preceding claim, wherein the device is configured to enable orientation of the acetabular component relative to the subject to be controlled based on an acetabular centre edge angle measured from the subject.

9. A device according to any of claims 1 to 8, wherein the device further comprises at least one guide portion to define at least an orientation relative to the subject when the device is installed to which an acetabular component can be installed to.

10. A device according to any of claims 22 to 24, wherein the guide portion comprises at least one pin placement guide (144, 145) or an elongate rod (144a, 145a, 318).

11. A device according to any of claims 2 to 10, wherein the position of the superior locator (112, 212, 312) is adjustable by the user relative to the device body, the superior locator preferably being pivotably adjustable relative to the device.

12. A device according to claim 11, wherein the position of the superior locator (112, 212, 312) relative to the device body can be selected by the user based on the centre edge angle measured from the subject.

13. A device according to any preceding claim, wherein the device further comprises means (130, 230) for substantially defining the centre of a subject's acetabulum.

14. A device according to any preceding claim, wherein the first locator and/or second locator and/or third locator are each configured to substantially make point-contact with their respective mounting location when the device is installed.

15. A device according to any of claims 2 to 14, the device having an acetabular alignment axis that orients when installed to at least an orientation to which the acetabular component is to be aligned when the acetabular component is installed, an angle C between the superior locator (112, 212, 312) and posterior locator ((123, 223, 323) as measured about the acetabular alignment axis in a plane perpendicular to the acetabular alignment axis, being about 66 to 86°, preferably about 74 to 78°, more preferably about 76°, the device preferably being configured such that the alignment reference element (140, 240, 340) is to be aligned with a natural landmark comprising a bony prominence in the obturator externus tendon groove of a subject and wherein an angle A between a first vector between the subject's acetabular centre and the superior locator (112, 212, 312) and a second vector between the subject's acetabular centre and the natural landmark, when the first and second vectors are projected on the sagittal plane, is about 112 to 132°, preferably about 120 to 124°, more preferably about 122° as measured when the device is installed.

16. A kit of parts for providing, when assembled, a device according to any preceding claim.

## Patentansprüche

1. Vorrichtung (100, 200, 300) zur Verwendung beim Erleichtern der Ausrichtung einer Hüftgelenkpfannenkomponente in Bezug auf das Becken einer Person, wobei die Vorrichtung einen Vorrichtungskörper und mindestens einen ersten Lokator (112, 212, 312) zur Platzierung auf einem ersten vorbestimmten Befestigungsort auf dem Hüftgelenkpfannenrand einer Person, einen zweiten Lokator (122, 222, 322) zur Platzierung auf einem zweiten vorbestimmten Befestigungsort auf dem Hüftgelenkpfannenrand und einen dritten Lokator (123, 223, 323) zur Platzierung auf einem dritten vorbestimmten Befestigungsort auf dem Hüftgelenkpfannenrand aufweist, wobei die Vorrichtung eine Ausrichtung und/oder Position in Bezug auf die Person definiert, wenn sie installiert ist, um die Ausrichtung einer Hüftgelenkpfannenkomponente zu erleichtern, **dadurch gekennzeichnet, dass** die Vorrichtung ferner ein Ausrichtungsbezugselement (140, 240, 340) zum Ausrichten nach einem vorbestimmten natürlichen Orientierungspunkt der verwendeten Person umfasst, wobei der natürlich Orientierungspunkt von dem Nutzer identifizierbar ist, der erste, zweite und dritte Lokator in Bezug auf die Ausrichtung des Bezugselements (140, 240, 340) so platziert ist, dass bei der Ausrichtung des Ausrichtungsbezugselement nach dem natürlichen Orientierungspunkt die Lokatoren ihre jeweiligen Befestigungsorte auffinden, wobei der natürliche Orientierungspunkt ein vorbestimmter Orientierungspunkt ist, der ein anderer als der Hüftgelenkpfannenrand ist.

2. Vorrichtung nach Anspruch 1, wobei der Lokator oder einer der Lokatoren ein höherer Lokator (112, 212, 312) zur Platzierung auf einem vorbestimmten Befestigungsort auf dem höheren Hüftgelenkpfannenrand, einer ein vorderer Lokator (122, 222, 322) zur Platzierung auf einem vorbestimmten Befestigungsort auf dem vorderen Hüftgelenkpfannenrand und einer ein hinterer Lokator (123, 223, 323) zur Platzierung auf einem vorbestimmten Befestigungsort auf dem hinteren Hüftgelenkpfannenrand ist.

3. Vorrichtung nach Anspruch 1, wobei das Ausrichtungsbezugselement eine Stiftplatzierungsführung (140) zum Führen der Platzierung eines Stifts in dem Becken der Person an dem natürlichen Orientierungspunkt umfasst oder es eine Ausrichtungsmarkierung (240, 340) umfasst, die nach dem verwendeten natürlichen Orientierungspunkt ausgerichtet werden kann.

4. Vorrichtung nach einem der vorhergehenden Ansprüche, wobei die Vorrichtung so ausgelegt ist, dass das Ausrichtungsbezugselement (140, 240, 340) nach einem knöchernen Vorsprung in der Sehnenvertiefung des äußeren Hüftlochmuskels einer Person ausgerichtet werden muss.

5. Vorrichtung nach einem der Ansprüche 2 bis 4, wobei der Abstand zwischen dem vorderen (122) und dem hinteren Lokator (123) durch den Nutzer anpassbar ist.

6. Vorrichtung nach einem der Ansprüche 2 bis 5, wobei der Vorrichtungskörper einen ersten (110, 210) und einen zweiten Abschnitt (120, 220) aufweist, wobei der höhere Lokator (112, 212) auf dem ersten Abschnitt des verwendeten Vorrichtungskörpers oder damit gekoppelt ist und der vordere und der hintere Lokator auf dem zweiten verwendeten Abschnitt oder damit gekoppelt ist, wobei der zweite Abschnitt verschiebbar an dem ersten verwendeten Abschnitt befestigt ist.

7. Vorrichtung nach einem der Ansprüche 2, 5 oder 6, wobei der vordere Lokator (122, 222, 322) im Wesentlichen diametral gegenüber des hinteren Lokators (123, 223, 323) liegt.

8. Vorrichtung nach einem der vorhergehenden Ansprüche, wobei die Vorrichtung dazu ausgelegt ist, die Ausrichtung der Hüftgelenkpfannenkomponente in Bezug auf die zu steuernde Person basierend auf einem mittleren Hüftgelenkpfannenkantenwinkel von der Person zu ermöglichen.

9. Vorrichtung nach einem der Ansprüche 1 bis 8, wobei die Vorrichtung ferner mindestens einen Führungsabschnitt umfasst, um mindestens eine Ausrichtung in Bezug auf die Person zu definieren, wenn die Vorrichtung installiert ist, an der eine Hüftgelenkpfannenkomponente installiert werden kann.

10. Vorrichtung nach einem der Ansprüche 22 bis 24, wobei der Führungsabschnitt mindestens eine Stiftplatzierungsführung (144, 145) oder einen länglichen Stab (144a, 145a, 318) umfasst.

11. Vorrichtung nach einem der Ansprüche 2 bis 10, wobei die Position des höheren Lokators (112, 212, 312) durch den Nutzer in Bezug auf den Vorrichtungskörper anpassbar ist, wobei der höhere Lokator vorzugsweise in Bezug auf die Vorrichtung schwenkbar anpassbar ist.

12. Vorrichtung nach Anspruch 11, wobei die Position des höheren Lokators (112, 212, 312) in Bezug auf den Vorrichtungskörper durch den Nutzer basierend auf dem mittleren Kantenwinkel, der von der Person gemessen wird, ausgewählt werden kann.

13. Vorrichtung nach einem der vorhergehenden Ansprüche, wobei die Vorrichtung ferner Mittel (130, 230) zum im Wesentlichen Definieren der Mitte der Hüftgelenkpfanne einer Person umfasst.

14. Vorrichtung nach einem der vorhergehenden Ansprüche, wobei der erste Lokator und/oder zweite Lokator und/oder dritte Lokator jeweils dazu ausgelegt sind, im Wesentlichen einen Punktkontakt mit ihrem jeweiligen Befestigungsort herzustellen, wenn die Vorrichtung installiert ist.

15. Vorrichtung nach einem der Ansprüche 2 bis 14, wobei die Vorrichtung eine Hüftgelenkpfannenausrichtungsachse aufweist, die, wenn sie installiert ist, nach mindestens einer Ausrichtung ausrichtet, nach der die Hüftgelenkpfannenkomponente ausgerichtet werden muss, wenn die Hüftgelenkpfannenkomponente installiert ist, einem Winkel C zwischen dem höheren Lokator (112, 212, 312) und hinteren Lokator (123, 223, 323), gemessen um die Hüftgelenkpfannenausrichtungsachse in einer Ebene senkrecht zu der Hüftgelenkpfannenausrichtungsachse, der etwa 66 bis 86°, vorzugsweise etwa 74 bis 78°, besonders vorzugsweise etwa 76° beträgt, wobei die Vorrichtung vorzugsweise ausgelegt ist, sodass das Ausrichtungsbezugselement (140, 240, 340) nach einem natürlichen Orientierungspunkt ausgerichtet werden muss, das einen knöchernen Vorsprung in der Sehnenvertiefung des äußeren Hüftlochmuskels der Person umfasst und wobei ein Winkel A zwischen einem ersten Vektor zwischen der Hüftgelenkpfannenmitte der Person und dem höheren Lokator (112, 212, 312) und einem zweiten Vektor zwischen der Hüftgelenkpfannenmitte der Person und dem natürlichen Orientierungspunkt, wenn der erste und der zweite Vektor auf die Sagittalebene projiziert sind, etwa 112 bis 132°, vorzugsweise etwa 120 bis 124°, besonders vorzugsweise etwa 122° beträgt, wie gemessen wird, wenn die Vorrichtung installiert ist.

16. Teilesatz zum Bereitstellen einer Vorrichtung nach einem der vorhergehenden Ansprüche, wenn diese zusammengesetzt ist.

## Revendications

1. Dispositif (100, 200, 300) utilisé pour faciliter l'alignement d'un composant acétabulaire relativement au bassin d'un sujet, le dispositif comprenant un corps de dispositif, et au moins un premier dispositif de localisation (112, 212, 312) pour le positionnement dans un premier emplacement de montage prédéterminé sur le rebord acétabulaire d'un sujet, un deuxième dispositif de localisation (122, 222, 322) pour le positionnement dans un deuxième emplacement de montage prédéterminé sur le rebord acétabulaire, et un troisième dispositif de localisation (123, 223, 323) pour le positionnement dans un troisième emplacement de montage prédéterminé sur le rebord acétabulaire, le dispositif définissant une orientation et/ou une position relativement audit sujet lors de l'installation pour faciliter l'alignement d'un composant acétabulaire, **caractérisé en ce que** le dispositif comprend en outre un élément de référence d'alignement (140, 240, 340) pour l'alignement avec un repère naturel prédéterminé du sujet en cours d'usage, le repère naturel étant identifiable par l'utilisateur, les premier, deuxième et troisième dispositifs de localisation étant situés relativement à l'élément de référence d'alignement (140, 240, 340), de sorte que lors de l'alignement de l'élément de référence d'alignement avec le repère naturel, les dispositifs de localisation se placent dans leur emplacement de montage respectif, le repère naturel étant un repère prédéterminé autrement que sur le rebord acétabulaire.

2. Dispositif selon la revendication 1, ledit dispositif de localisation ou un desdits dispositifs de localisation étant un dispositif de localisation supérieur (112,212,312) pour le positionnement dans un emplacement de montage prédéterminé sur le rebord acétabulaire supérieur, dont un est dispositif de localisation antérieur (122, 222, 322) pour le positionnement dans un emplacement de montage prédéterminé sur le rebord acétabulaire antérieur, et un est dispositif de localisation postérieur (123, 223, 323) pour le positionnement dans un emplacement de montage prédéterminé sur le rebord acétabulaire postérieur.

3. Dispositif selon la revendication 1, l'élément de référence d'alignement comprenant soit un guide de positionnement de broche (140) guidant le positionnement d'une broche dans le bassin du sujet au repère naturel, soit un repère d'alignement (240, 340) pouvant être aligné avec le repère naturel utilisé.

4. Dispositif selon une quelconque des revendications précédentes, le dispositif étant configuré de sorte que l'élément de référence d'alignement (140, 240, 340) soit aligné avec une proéminence osseuse dans la rainure du tendon du muscle obturateur externe d'un sujet.

5. Dispositif selon une quelconque des revendications 2 à 4, l'espacement entre les dispositifs de localisation antérieur (122) et postérieur (123) étant ajustable par l'utilisateur.

6. Dispositif selon une quelconque des revendications 2 à 5, le corps du dispositif possédant des première (110, 210) et deuxième (120, 220) parties, le dispositif de localisation supérieur (112, 212) se trouvant sur la première partie du corps de dispositif en cours d'utilisation, ou couplé sur celui-ci, et les dispositifs de localisation antérieur et postérieur se trouvant sur la deuxième partie utilisée, ou couplés sur celle-ci, la deuxième partie étant montée par coulissement sur la première partie en cours d'utilisation.

7. Dispositif selon une quelconque des revendications 2, 5 ou 6, le dispositif de localisation antérieur (122, 222, 322) étant situé substantiellement de façon diamétralement opposée au dispositif de localisation postérieur (123, 223, 323).

8. Dispositif selon une quelconque des revendications précédentes, le dispositif étant configuré de façon à permettre l'orientation du composant acétabulaire relativement au sujet à contrôler, sur la base d'un angle de bord central acétabulaire mesuré depuis le sujet.

9. Dispositif selon une quelconque des revendications 1 à 8, le dispositif comprenant en outre au moins une partie de guide pour définir au moins une orientation relative au sujet lorsque le dispositif est installé, sur lequel un composant acétabulaire peut être installé.

10. Dispositif selon une quelconque des revendications 22 à 24, la partie de guide comprenant au moins un guide de positionnement de broche (144, 145) ou une tige allongée (144a, 145a, 318).

11. Dispositif selon une quelconque des revendications 2 à 10, la position du dispositif de localisation supérieur (112, 212, 312) pouvant être ajustée par l'utilisateur relativement au corps du dispositif, le dispositif de localisation supérieur s'ajustant de préférence par pivotement relativement au dispositif.

12. Dispositif selon la revendication 11, la position du dispositif de localisation supérieur (112, 212, 312) relativement au corps du dispositif pouvant être sélectionnée par l'utilisateur sur la base de l'angle de bord central mesuré depuis le sujet.

13. Dispositif selon une quelconque des revendications précédentes, le dispositif comprenant en outre un élément (130, 230) pour définir substantiellement le centre de l'acétabulum d'un sujet.

14. Dispositif selon une quelconque des revendications précédentes, le premier dispositif de localisation et/ou le deuxième dispositif de localisation et/ou le troisième dispositif de localisation étant chacun configuré pour effectuer substantiellement un contact ponctuel avec leur position de montage respective lorsque le dispositif est installé.

15. Dispositif selon une quelconque des revendications 2 à 14, le dispositif présentant un axe d'alignement acétabulaire orientant, lorsqu'il est installé, vers au moins une orientation dans laquelle le composant acétabulaire doit être aligné lorsque le composant acétabulaire est installé, un angle C entre le dispositif de localisation supérieur (112, 212, 312) et le dispositif de localisation postérieur (123, 223, 323) mesuré autour de l'axe d'alignement acétabulaire dans un plan perpendiculaire à l'axe d'alignement acétabulaire, mesurant environ 66° à 86°, de préférence 74° à 78°, mieux encore environ 76°, le dispositif étant configuré de préférence de sorte que l'élément de référence d'alignement (140, 240, 340) soit aligné avec un repère naturel comprenant une proéminence osseuse dans la rainure du tendon du muscle obturateur externe d'un sujet, et un angle A entre un premier vecteur entre le centre acétabulaire du sujet et le dispositif de localisation supérieur (112, 212, 312) et un deuxième vecteur entre le centre acétabulaire du sujet et le repère naturel, lorsque les premier et deuxième vecteurs sont projetés dans le plan sagittal, mesurant environ de 112° à 132°, de préférence environ de 120° à 124°, mieux encore environ 122° mesuré lorsque le dispositif est installé.

16. Kit de pièces permettant d'obtenir, lorsqu'il est assemblé, un dispositif selon une quelconque des revendications précédentes.
